# EUROPEAN PATENT SPECIFICATION

(11) **EP 1 490 069 B1**
(45) Date of publication and mention of the grant of the patent: **15.10.2008**
(21) Application number: 03718054.4
(22) Date of filing: 21.03.2003
(51) Int. Cl.: A61K 31/56, A61P 3/10, A61P 9/10, A61P 29/00, A61P 37/02

(54) **ALPHA AED AND BETA AED REGULATION OF NUCLEAR TRANSCRIPTION, GENE REGULATION, AND/OR GENE EXPRESSION**
ALPHA-AED UND BETA-AED-REGULIERUNG DER NUKLEARTRANSKRIPTION, GENREGULIERUNG UND/ODER GENEXPRESSION
REGULATION DE TRANSCRIPTION NUCLEAIRE, REGULATION GENIQUE, ET/OU D'EXPRESSION GENIQUE AU MOYEN DE ALPHA AED ET DE BETA AED

(30) Priority: 21.03.2002 US 365817 P
(43) Date of publication of application: 29.12.2004
(73) Proprietor: LORIA, Roger M., Richmond, VA 23227 (US)
(72) Inventor: LORIA, Roger M., Richmond, VA 23227 (US)
(74) Representative: Couchman, Jonathan Hugh
(86) International application number: PCT/US2003/009141
(87) International publication number: WO 2003/080810

(56) References cited:
- WO-A-93/20696
- WO-A2-02/072003
- US-A- 5 641 768
- US-A1- 2001 014 675
- WAXMAN D J: "Role of metabolism in the activation of dehydroepiandrosterone as a peroxisome proliferator" JOURNAL OF ENDOCRINOLOGY, BRISTOL, GB, vol. 150, 1996, pages S129-S147, XP002954541 ISSN: 0022-0795

## Description

### FIELD OF THE INVENTION

The present invention relates to the regulation of nuclear transcription using αAED and βAED.

### BACKGROUND OF THE INVENTION

Peroxisome Proliferator Activated Receptors (PPARs) are a Subclass of nuclear hormone transcription factors that have tissue-specific distribution and regulate gene expression. Three major subtypes of PPARs have been described: α, γ, and δ. The PPARs were originally thought to be exclusively linked to the control of lipid metabolism and homeostasis. However, studies have revealed that PPAR activation can influence a wide range of biologic activities including cellular proliferation, differentiation and apoptosis. This receptor family is implicated in a wide range of human conditions including, but not limited to, obesity, diabetes, atherosclerosis, inflammation, cancer, and aging (Isemann and Green, Nature 347:645-650 (1990); Collingwood et al, J. Mol. Endocrinol. 23:255-275 (1999); McKenna et al, Endocr. Rev. 20:321-344 (1999); Schoonjans et al, Curr. Opin. Lipidol. 8:159-166 (1997); and Greene et al, Prostaglandins & Other Lipid Mediators 62:45-73 (2000)),

PPARγ and retinoid receptor alpha form a heterodimer, which in the presence of ligand, binds to DNA response elements that help regulate expression of target genes (Mangelsdorf and Evans, Cell 83:841-850 (1995)). The mouse steroid receptor coactivator -1 (mSRC-1), has been cloned and has been found to interact with PPARγ and to have a role in enhancing ligand-dependent transcription (Zhu et al, Gene Expr. 6(3):185-195 (1996); and Jain et al, Am. J. Pathol. 153:349-354 (1998)). PPARγ also functions as a master regulator of adipogenesis (Li et al, Mol. Gen Metabol. 70:159-161 (2000); Tontonoz and Spiegelman, Cell 79:1147-1156 (1994); and Kliewer et al, Cell 83:813-819 (1995)). Previous studies have shown that PPARγ is activated by selected prostaglandins, prostaglandin-like molecules, and arachidonic acid metabolites (Dussault and Forman, Prostaglandins & Other Lipid Mediators 62:1-13 (2000); Kirk et al, Prostaglandins & Other Lipid Mediators 62:15-21 (2000); and Bishop-Bailey and Hiat, J. Biol. Chem. 274:17042-17048 (1999)).

Prostaglandins of the A and J series in particular cause tumor cell apoptosis (McClay, Prostaglandins & Other Lipid Mediators 62:75-90 (2000); Clay et al, Prostaglandins & Other Lipid Mediators 62:23-32 (2000); and Kubota et al, Cancer Res. 58:3344-3352 (1998)) and can also regulate endothelial cell function by inducing heat shock proteins (Koizumi et al, Prostaglandins 43:121-131 (1992); and Maggi et al, Diabetes 49:346-355 (2000)). It is particularly relevant that 15 deoxy Δ ^{12,14} prostaglandins J2 induce endothelial cell apoptosis via a PPAR γ dependent pathway (Bishop-Bailey and Hiat, J. Biol. Chem. 274:17042-17048 (1999); McClay, Prostaglandins & Other Lipid Mediators 62:75-90 (2000); and Clay et al, Prostaglandins & Other Lipid Mediators 62:23-32 (2000)). PPARγ is activated by a number of non-steroidal anti-inflammatory drugs, such as indomethacin (Lehmann et al, J. Biol. Chem. 270:12953-12956 (1995)) and the anti-diabetic drug thiazolidinediones, and clearly has a role in control of inflammation.

All of the commonly occurring prostanoids (prostaglandins) are formed via the cyclooxygenase (COX; a.k.a. prostaglandin endoperoxide H synthases (PGHS)) enzymatic pathway (Smith et al, J. Biol. Chem. 271:33157-33160 (1996)). COX is the key regulatory enzyme of the prostaglandin/eicosanoid pathway leading to the conversion of arachidonic acid to prostanoids and thromboxanes (Willoughby et al, Lancet 355 (9204):646-648 (2000); Colville-Nash and Gilroy, Prostaglandins Other Lipid Mediat. 62:33-43 (2000); Berg et al, J. Immuno/. 166:2674-2680 (2001); and William et al, J. Biol. Chem. 271:33157-33160 (1996)). COX is known to exist in at least two isoforms: a constitutively expressed COX-1 and a mitogen/cytokine induced isoform COX-2.

The promotor /enhancer regions of COX-2 contain sequences specific for the binding of various transcription factors including NF-κB (nuclear factor-κB), which is involved in rapid up-regulation of inflammatory molecules (Jobin et al, Immunol. 95:537-543 (1998)). NF-κB has a pivotal role in chronic inflammatory diseases (Barnes et al, N. Engl. J. Med 336:1066-1071 (1997)). As stated by Lentsch and Ward (Lentsch and Ward, Clin. Chem. Lab. Med. 37:205-208 (1999)), under normal conditions, NF-κB is localized to the cell cytosol by inhibitory proteins of the IκB family. The inflammatory response leads to the degradation of these IκB proteins, resulting in the release of NF-κB which is translocated to the cell nucleus where it initiates transcription. This activation of NF-κB results in the production of proinflammatory mediators that facilitate inflammatory tissue injury. The suppression of NF-κB consequently will be beneficial to minimize or reduce inflammation injury.

Increases in COX-2, NF-κB and prostaglandin, all function to amplify the inflammatory process. Yu et al. (Yu et al, Inflamm. Res. 47:167-173 (1998)) reported that the proinflammatory effects of IL-13 are mediated in part by its ability to increase prostaglandin E2 levels in normal human polymorphonuclear neutrophils (PMN) and to enhance the expression of COX-2. These results suggest that IL-13 can also modulate the inflammatory reactions via the cyclooxygenase pathyway.

Inflammatory events have also been shown to be associated with plaque formation in the brains of patients with Alzheimer's disease (AD). Both cyclooxygenases and PPAR γ levels are significantly elevated in the brains of Alzheimer's patients (Kitamura et al, Biochem. Biophys. Res. Commun. 254:582-586 (1999)). Previous studies have shown that PPAR γ activators have inhibitory effects on the inflammatory events in AD brains.

In addition, reports show that PPARγ mediates apoptosis of human monocyte-derived macrophages (Chinetti et al, J. Biol. Chem. 273:25573-25580 (1998)), human gastric cancer cells (Takahashi et al, FEBS Lett. 455:135-139 (1999)) and human breast cancer cells *in vitro* and in BNX mice *in vivo* (Elstner et al, Proc. Natl. Acad. Sci. U.S.A. 95:8806-8811 (1998)). It is particularly relevant that 15 deoxy Δ ^{12,14} prostaglandins (15-deoxy-Δ^{12,14} PGJ₂) induce endothelial cell apoptosis via a PPARγ dependent pathway.

PPARγ has a potent antitumor effect against human prostate cancer, both in vitro and in vivo (Kubota et al, Cancer Res. 58:3344-3352 (1998)), and is a potent inhibitor of angiogenesis in vitro and in vivo (Xin et al, J. Biol. Chem. 274:9116-9121 (1999)). The expression of PPARγ messenger RNA and protein in several primary human cancers and cancer cell lines is documented. McClay (Prostaglandins & Other Lipid Mediators 62:75-90 (2000)) describes 15-deoxy-Δ^{12,14} PGJ₂ cytotoxicity in different cancer types, which are mediated by PPARγ activation

Several studies have implicated 15-deoxy-Δ^{12,14} PGJ₂ activation of PPARγ in the apoptosis of monocytes and macrophages and raised the possibility of using PPARγ as a possible therapeutic target for inflammation and atherosclerosis. For example, Chinetti and colleagues found that 15-deoxy-Δ^{12,14} PGJ₂ activates PPARγ and induces caspase-mediated apoptosis of human macrophages and monocytes. This may potentially occur by antagonizing AP-1, STAT, and NF-κB inhibiting inducible nitric oxide synthetase, gelatinase B and scavenger receptor genes and by suppression of inflammatory cytokines.

PPARγ activation may affect the roles of erythroid, myeloid, monocytic, T and B lymphocytic and endothelial cell function during the immune response, particularly in modulation of acute versus chronic responses, and the duration of chronic responses.

Activation of PPARγ using thiazolidinediones induces adipogenesis of fibroblasts and differentiation of liposarcoma cells. Knockout studies in mice have shown that PPARγ is essential for differentiation of the placenta, heart, and adipose tissue. In particular, it causes adipocyte differentiation and appears to be a key in mediating fat storage. A mutation in PPARγ2 (Pro12Ala) is associated with the early onset of extreme obesity and reduced fasting glucose tolerance PPARγ is also associated with mesenteric fat hypertrophy in Crohn's disease (Desreumx et al, Gastroenterology 117(1):73-81 (1999)).

Waxman (J. Endocrinology Bristol, GB, vol. 150, 1996, pages 5129-5147) describes the role of metabolism in the activation of dehydroepiandrosterone as a peroxisome proliferator.

### SUMMARY OF THE INVENTION

Δ5-Androstenediol (AED) is a naturally-occurring metabolite of dehydroepiandrosterone (DHEA), the most abundant product of the adrenal glands. AED may also arise from the metabolism of other steroids. AED exists in two epimeric forms: Δ5-Androstene-3β,17α-diol (αAED) and Δ5-Androstene-3β,17β-diol (βAED). Both are naturally occurring metabolites of dehydroepiandrosterone (DHEA). Until recently, however, the various beneficial effects of these respective epimers have not been appreciated. Until now, there has been no established relationship between either of those epimers and COX-2, NF-κB and PPARγ regulation.

The present invention relates to the regulation of nuclear transcription and/or gene regulation in a patient in need of such regulation. This nuclear and/or gene regulation can be accomplished through regulation of the PPAR system, including PPAR γ, PPAR α and/or PPAR δ.

The present invention also relates to regulation of the activity or amount of PPAR-γ, COX-2 and/or NFκB in a patient in need of such regulation. In some embodiments, one or more compounds of Δ5-Androstene-3β,17α-diol (αAED), also referred to herein as formula (I), up-regulates said activity or amount of PPAR-γ, COX-2, and/or NFκB. In other embodiments, one or more compounds of Δ5-Androstene-3β,17β-diol (βAED), also referred to herein as formula (II), down-regulates said activity or amount of PPAR-γ and/or NFκB, and/or up regulates said activity or amount of COX-2. Further, one or more compounds of formula (I) and formula (II) can be delivered to the tissues of said patient or administered to said patient sequentially, such that said activity or amount of PPAR-γ, COX-2, and/or NFκB is down regulated, or alternately, such that said activity or amount of PPAR-γ, COX-2, and/or NFκB is up regulated.

The invention provides compounds for regulating one or more of the conditions selected from the group consisting of adipogenesis, angiogenesis, mesenteric fat hypertrophy, atherosclerosis, and Alzeimer's disease in a patient in need of such regulation. These conditions may be regulated through the regulation of nuclear transcription and/or gene regulation. This nuclear and/or gene regulation can be accomplished through regulation of the PPAR system, including PPAR γ, PPAR α and/or PPAR δ.

According to the present invention, regulation of these conditions is achieved by delivering to the tissues of said patient or administering to said patient a therapeutically effective amount of one or more compounds of formula (I):

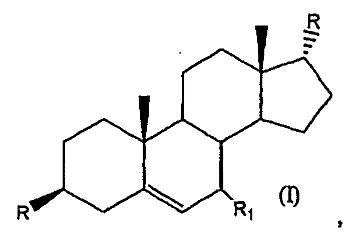

or delivering to the tissues of said patient or administering to said patient a therapeutically effective amount of one or more compounds of formula (II):

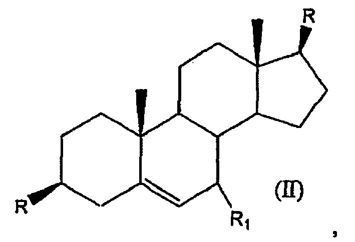

or delivering to the tissues of said patient or administering to said patient a therapeutically effective amount of one or more compounds of formula (I) and formula (II);

wherein each R₁ is independently selected from the group consisting of H and R;

wherein each R is independently selected from the group consisting of a hydroxyl, a protected hydroxyl, a C₁-C₃₀ ether and a C₁-C₃₀ ester.
In embodiments, each R of the formula (I) and/or formula (II) compounds is-OH and/or each R₁ is -H.

These compounds are independently administered orally, topically, subcutaneously, parenterally, transdermally, mucosally, rectally, intranasally, via inhalation, via insufflation, via a patch, via application to the site of the tumor or tumor bed, via installation into a wound, by buccal, or by sublingual administration. In some embodiments, one or more compounds of formula (II) are delivered to the tissues of said patient or administered to said patient after said one or more compounds of formula (I). In other embodiments one or more compounds of formula (I) are delivered to the tissues of said patient or administered to said patient after said one or more compounds of formula (II). In each of these embodiments, the time that passes between the delivery or administration to the patient affects the regulation of PPAR-γ, COX-2, and/or NFκB.

### DETAILED DESCRIPTION OF THE INVENTION

The inventor unexpectedly found that αAED and βAED, and their analogues, are able to regulate nuclear transcription and/or gene expression and gene regulation, in particular nuclear transcription and/or gene expression controlled by PPAR. Thus, the present invention provides methods for regulating nuclear transcription, in particular nuclear transcription controlled by PPAR.

The inventor also unexpectedly found that αAED and βAED are able to regulate the level of the nuclear receptor, PPARγ. αAED elevates and βAED reduces the levels of PPARγ. The sequential combination of both αAED and βAED within a specific period of time also reduces the levels of PPARγ. These reduced levels of PPARγ are often lower than reduction using βAED alone, thus during this specific period of time, αAED and βAED work as a type of synergistic agents. This period of time must be long enough for αAED and βAED to achieve a desired net effect that is different from the effect of combining αAED and βAED simultaneously or almost simultaneously (within 1, 2, 4, or 8 hours); however, the period must also be short enough to prevent αAED and βAED from working as stabilizing agents instead of synergistic agents. If the time period is long enough to allow αAED and βAED to work as stabilizing agents, αAED can be administered after βAED to up-regulate the effects βAED in order to establish homeostasis and vice versa. In order to establish homeostasis, the sequential combination of αAED and βAED in a sufficient period of time can be repeated until the desired result is achieved. The expression, or turn off, of many genes are controlled by PPARγ, and thus the expression/regulation of these genes can be regulated, up or down, by the levels of αAED and βAED. By regulating these various genes, αAED and βAED can be used to regulate adipogenesis or angiogenesis.

For example, PPARγ induces adipogenesis. Therefore, if one would like to reduce adipogenesis, one of skill in the art would administer to a patient either βAED alone or in combination with αAED. Furthermore, if one would like to reduce angiogenesis, one of skill in the art would administer to a patient either βAED alone or in combination with αAED.

Thus, the present invention relates to the regulation of nuclear transcription controlled by PPARγ. The present invention also relates to the treatment or prophylaxis in, a patient suffering from, or at risk of, of adipogenesis or of angiogenis comprising, administering to said patient a therapeutically effective, or prophylactically effective, amount of αAED alone or αAED sequentially together with βAED.

The inventor also unexpectedly found that αAED and βAED can regulate the levels of COX-2 and NF-κB. It was discovered that αAED increases the levels of COX-2, whereas βAED results in only a mild elevation of COX-2. In addition, the inventor found that sequential administration of αAED and βAED over specific period of time reduces the levels of COX-2 and is anti-inflammatory. This period of time must be long enough for αAED and βAED to achieve a desired net effect on COX-2 that is different from the effect of combining αAED and βAED almost simultaneously and short enough to prevent αAED and βAED from working independently to elevate COX-2 levels. Furthermore, the inventor found that αAED increases the levels of the proinflammatory nuclear factor, NF-κB, whereas βAED reduces the levels of NF-κB. In some instances, an increase in the levels of NF-κB is desirable, for instance a small inflammatory burst is often needed to initiate a CTL-related immune response.

αAED and βAED may act directly on COX-2 and NF-κB, or may act indirectly by regulating the levels of PPARγ and/or prostaglandins, which would then regulate the levels of COX-2 and NF-κB (Gupta et al, J. Biol. Chem. 276(33):31059-31066 (2001); Gupta et al, J. Biol. Chem. 276(32):29681-29687 (2001); and Subbaramaiah et al, J. Biol. Chem. 276:12440-12448 (2001)).

By regulating the amounts of COX-2 and NF-κB, one can treat Alzheimer's disease.

Thus, the present invention provides a medicament for treating, effecting, or ameliorating prophylaxis in, a patient suffering from, or at risk of, Alzheimer's disease wherein a therapeutically effective, or prophylactically effective, amount of αAED, βAED, or a combination thereof is administrated to said patient.

### Definitions:

The following definitions are provided in order to facilitate the understanding of the instant invention.

As used herein, and unless stated otherwise, the term "patient" refers to any animal, particularly mammal, e.g., human, bovine, equine, canine, feline, rodent, or pr imate.

As used herein, and unless stated otherwise, the term "AED analogue" contemplates AED wherein one or both of the hydroxy substituents is modified with a protecting group, or is modified to a pharmaceutically acceptable salt, such as a halide or sulphate salt. AED analogues useful in the methods of the instant application can be identified by comparing the properties of the analogues with the properties of αAED and βAED described herein. Useful analogues will show activities similar to, or preferably better than, one or more of the activities observed with either αAED or βAED. These activities include, but are not limited to, modulation of PPAR γ, COX-2, and/or NF-κB activities and/or amounts.

Examples of methods that can be used to characterize useful analogues can be found, for instance, in Example 1, herein- Additional animal models can also be used to characterize useful analogues, such as, for example, the mouse models for inflammatory bowel disease using DNBS or TNBS and the mouse or rat models of obesity and diabetes (db/db mice, ob/ob mice or Zucher rat). Following treatment with analogues, for example, activities and/or amounts of PPAR γ, COX-2, and/or NP-κB can be determined in a selection of tissues (including blood, colon, intestine, liver, and fat, for example) by Western blot or ELISA by methods well-known in the art.

As used herein, and unless stated otherwise, the term activities "similar to" or the like, means a quantitative measurement that is not necessarily statistically significant from the measurement obtained when either αAED or βAED are used in the assay. Alternatively, "similar to" can include values that are at least 1%, 2%, 5%, 7%, 10%, 15%, 20%, 25%, 30%, 40%, or 50% higher or lower than that observed with either αAED or βAED.

As used herein, and unless stated otherwise, the term activities "better than" or the like, means a quantitative measurement that is typically statistically significant from the measurement obtained when either αAED or βAED are used in the same assay. Depending on the assay and on the desired result, this may be a value that is either higher or lower than the value obtained when either αAED or βAED are used in the assay. The higher or lower value may be at least a 5%, 7%, 10%, 15%, 20%, 25%, 30%, 40%, 50%, 100%, 200% or greater change.

As used herein, and unless stated otherwise, the term "modulates" or "modulation" means a quantitative measurement of the activities and/or amounts of PPAR γ, COX-2, and/or NF-κB that is typically either statistically different or detectably different from the measurement obtained when αAED or βAED or both (together or sequentially) or analogues thereof are used in the same assay or the same patient. Depending on the assay and on the desired result, this may be a value that is either higher or lower than the value obtained when neither αAED or βAED are used in the assay. The higher or lower value may be an increase or a decrease of at least a 5%, 7%, 10%, 15%, 20%, 25%, 30%, 40%, 50%, 100%, 200% or greater change. Useful assays can be found in Example 1, herein and provided above under "examples of methods", along with many others well known in the art.

As used herein, and unless stated otherwise, the term "sequential" administration means not providing the two compounds at exactly the same time. The two compounds can be provided with a separation of about 24 hours, about 48 hours, about 96 hours or more. The precise schedule of administration will depend on the desired outcome in a particular disorder or disease as well as the exact analogues used. Useful assays to determine additional schedules of sequential administration can be found in Example 1, herein and provided above under "examples of methods", along with many others well known in the art.

As used herein, and unless stated otherwise, the term "at least about 96 hours" means a time that is about 96 hrs, for example 84 hrs, 90 hrs, 102, or 114 hrs, and including longer intervals, for example 120 hrs, 144 hrs, or 168 hrs or even substantially longer such as 1, 2 or more weeks, or 1, 2 or more months. In alternative embodiments, the term can be stated as at least 48 hrs, at least 72 hrs, at least 96 hrs, at least 120 hrs, at least 144 hrs, at least 168 hrs, at least 1 week, at least 2 weeks, or at least a month, for example. The term "about 96 hours" means a time that is about 96 hrs, for example 84 hrs, 90 hrs, 102, or 114 hrs, and including longer intervals, for example 120 hrs, 144 hrs, or 168 hrs, but not as long as a week or more. The term "about 48 hours" means a time that is about 48 hrs, for example, 36 hrs, 40 hrs, 50 hrs, 60 hrs, or 72 hrs. The term "about 24 hours" means a time that is about 24 hrs, for example, 20 hrs, 28 hrs, or 30 hrs. Unless stated otherwise, this time is measured from the time of administration of the first AED compound to a patient to the time when the second AED compound is administered.

As used herein, and unless stated otherwise, the term "alkyl" refers to a hydrocarbon containing up to about 20 carbon atoms in the form of normal, secondary, tertiary, cyclic, or mixed structures. Examples include, but are not limited to, -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, -CH₂CH₂CH₂CH₂CH₃, -CH(CH₃)CH₂CH₂CH₃, - CH(CH₂CH₃)₂, -C(CH₃)₂CH₂CH₃, -CH(CH₃)CH(CH₃)₂, -CH₂CH₂CH(CH₃)₂, -CH₂CH₂CH(CH₃)₂, -CH₂CH(CH₃)CH₂CH₃, -CH₂C(CH₃)₃, -CH₂CH₂CH₂CH₂CH₂CH₃, -CH(CH₃)CH₂CH₂CHCH₃, -CH(CH₂CH₃)(CH₂CH₂CH₃), -C(CH₃)₂CH₂CH₂CH₃, -CH(CH₃)CH(CH₃)CH₂CH₃, -CH(CH₃)CH₂CH(CH₃)₂, -C(CH₃)(CH₂CH₃)₂, -CH(CH₂CH₃)CH(CH₃)₂, -C(CH₃)₂CH(CH₃)₂, -CH(CH₃)C(CH₃)₃, cyclopropyl, cyclobutyl, cyclopropylmethyl, cyclopentyl, cyclobutylmethyl, 1-cyclopropyl-1-ethyl, 2-cyclopropyl-1-ethyl, cyclohexyl, cyclopentylmethyl, 1-cyclobutyl-1-ethyl, 2-cyclobutyl-1-ethyl, 1-cyclopropyl-1-propyl, 2-cyclopropyl-1-propyl, 3-cyclopropyl-1-propyl, 2-cyclopropyl-2-propyl, and 1-cyclopropyl-2-propyl. Further, the term alkyl contemplates saturated, unsaturated, and mixed or polyunsaturated systems, and thus the term includes alkanes, alkenes, alkynes, and combinations thereof.

As used herein, and unless stated otherwise, the term "organic moiety" means a hydrocarbon or mixed hydrocarbon moiety of up to about 15 carbons. Thus, the term contemplates, for example, alkyl, moieties such as a C₁₋₁₅ alkyl moiety, an aryl moiety such as phenyl, a mixed alkyl-aryl moiety such as a phenyl-C₁₋₄-alkyl moiety, or substituted hydrocarbons comprising, e.g., about one to about four substituents wherein the substituents are selected from functional groups comprising one or more heteroatoms (e.g., O, N, S, P, Se), halides, or other non-toxic moieties. Thus, for example, the hydrocarbon substituents may be independently chosen from among: -O-, -S-, -NR'- (including -NH-), -C(O)-, =O, =S, -N(R')₂- (including NH₂),-C(O)OR' (including -C(O)OH), -OC(O)R' (including -O-C(O)-H), -OR' (including-OH), -SR' (including -SH), -NO₂, -CN, -NHC(O)-, -C(O)NH-, -O-C(O)-, -C(O)-O-,-O-C₁₋₄ alkyl, -S-C₁₋₄ alkyl, -C(O)-C₁₋₄ alkyl, -O-C(O)-C₁₋₄ alkyl, -C(O)-O-C₁₋₄ alkyl, -C(O)-O-C₁₋₄, -N=, =N-OH, -OPO₃(R')₂, -OSO₃H₂, wherein each R' is selected from: H and an independently selected protecting group for the atom to which it is attached, and alkyl is C₁₋₈ alkyl, C₁₋₄ alkyl-aryl (e.g., benzyl), aryl, (e.g., phenyl) or C₁₋₄ alkyl-C₂₋₉ heterocycle. Substitutions are independently chosen. The substitutions listed above are substitutions that replace one or more carbon atoms, e.g., O or C(O), or one or more hydrogen atoms, e.g., -F, -Cl, -NH₂ or -OH.

As used herein, and unless stated otherwise, the term "treating" and the like means administering the compounds described in the instant application to a patient in need of such treatment and/or delivering the compound(s) to the patient's tissues. Patients in need of treatment can be identified by methods well-known to those of skill in the art, for example by care givers, preferably physicians, nurses, nurses assistants, etc.

As used herein, and unless stated otherwise, the term "prophylactically", "preventing" and the like means administering the compounds described in the instant application to a patient at risk of contracting a disease or medical condition described in the instant application, and where the condition's development or progression is slowed or halted or a symptom is improved.

As used herein, and unless stated otherwise, the term "at risk of contracting" means a patient who has been identified as being exposed to or as having one or more risk factors associated with the onset of disease. These risk factors may be environmental, genetic, or biological. For example, risk factors would include age of the patient or a family disposition to develop one of these conditions. Risk factors for the diseases described herein are known to one of ordinary skill in the art, such as physicians who typically diagnose and counsel patients.

As used herein, and unless stated otherwise, terms such as "ameliorate" or the like mean to reduce the severity, or to improve, or to mask one or more symptoms of the disease. For example, symptom or disease amelioration includes, but is not limited to, slowing or arresting tumor or tumor cell growth or reducing one or more symptoms such as pain, fever, or fatigue. This is a clinical determination within the sphere of the caregiver and the patient. Amelioration of symptoms may vary between sub-groups of patients depending on a variety of factors including but not limited to their genetic background.

As used herein, and unless stated otherwise, the term "effective amount" means an amount of compound that when administered ameliorates one or more symptoms or prevents the onset of one or more symptoms of the disease or is associated with a detectable change in molecules such as PPARγ, COX-2 and NF-κB. The effective amount for a particular patient and disease is a determination for the caregiver.

As used herein, and unless stated otherwise, the term "excipient" means a component or an ingredient that is compatible with the compounds of formula I and/or formula II and not overly deleterious to the patient or animal to which the formulation is to be administered. As used here, "excipients" include liquids, such as benzyl benzoate, cottonseed oil, N,N-dimethylacetamide, a C₂₋₁₂ alcohol (e.g., ethanol), glycerol, peanut oil, a polyethylene glycol (``PEG"), vitamin E, poppyseed oil, propylene glycol, safflower oil, sesame oil, soybean oil and vegetable oil. Excipients, as used herein will optionally exclude chloroform, dioxane, vegetable oil, DMSO or any combination of these. Excipients comprise one or more components typically used in the pharmaceutical formulation arts, e.g., fillers, binders, disintegrants and lubricants.

As used herein, and unless otherwise stated, the term "protecting group" means protecting groups such as hydroxyl groups, carboxyl groups, and amines include esters, alkyl ethers, alkenyl ethers, optionally substituted alkyl groups and optionally substituted alkenyl groups, e.g., acetate or propionate groups for hydroxyl groups and one or two independently selected methyl, ethyl, propyl or butyl groups for an amine group. The optional substitutions are as described for organic moieties defined above. Other protecting groups are described in "Protective Groups in Organic Chemistery", Theodora W. Greene (John Wiley & Sons, Inc. New York, 1991, ISBN'0-471-62301-6), incorporated herein by reference.

As used herein, and unless otherwise stated, term "regulates" or "regulation" means a quantitative measurement of the activities and/or amounts of PPAR γ COX-2, and/or NF-κB that is typically either detectably different or statistically different from the measurement obtained when αAED or βAED or both (together or sequentially) or analogues thereof are used in the same assay or the same patient. Depending on the assay and on the desired result, this may be a value that is either higher or lower than the value obtained when neither αAED nor βAED are used in the assay. The higher or lower value may be an increase or decrease of at least a 5%, 7%, 10%, 15%, 20%, 25%, 30%, 40%, 50%, 100%, 200% or greater change. Useful assays can be found in Example 1, herein and provided above under "examples of methods", along with many others well known in the art.

### Treatment Regimes

The invention includes treatment regimens comprising the administration of a pharmaceutical formulation comprising a therapeutically or prophylactically effective amount of one or more of αAED, analogues thereof, βAED, analogues thereof, or a combination thereof to a patient. The pharmaceutical formulation will further comprise conventional pharmaceutically acceptable excipients, diluents, carriers, and the like.

The active agents useful in the therapeutic regimens of the present invention are of the structure: 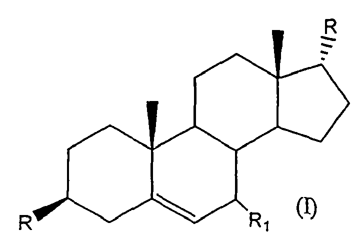 (analogs of αAED) and 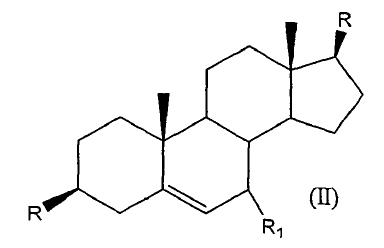 (analogs of βAED),
wherein each R₁ is H or R; and
wherein each R is independently selected from a hydroxyl, a protected hydroxyl, a C₁-C₃₀ ether and a C₁-C₃₀ ester;

Analogues of βAED and αAED having protecting groups (protected hydroxyl groups) can also be administered to the patient as a means of delivering αAED and/or βAED to target tissues. Acylation is a preferred method of protecting the compounds. Methods of making βAED, αAED, and analogues thereof are set forth in, e.g., U.S. Patent 5,387,513 (βAED) and U.S. Patent 2,521,586 (αAED). In some embodiments, R is-OH, or an -O- methyl keto radical to form an acetyl substituent. Similarly, analogues of αAED and βAED include pharmaceutically acceptable salts such as halides and sulphate salts.

When administered as described herein, these active agents can regulate the levels of PPAR-γ, thereby regulating adipogenesis, angiogenesis, mesenteric fat hypertrophy, and atherosclerosis. As noted above, αAED elevates and βAED reduces the levels of PPARγ. The combination of both αAED and βAED also reduce the levels of PPARγ if administered sequentially within a specific period of time. If a sufficient time period passes between administration of each of αAED and βAED, homeostasis at a desired level can be established. For example, PPAR γ is a key regulator of adipogenicity and controls the differentiation of fibroblast to adipocytes, which are phenotipically recognized by the lipid droplets. In a situation where there is excess adipogenicity, i.e. to much fat tissue, the sequential administration of αAED and βAED, would lead to a marked reduction in the levels of PPARγ, and a reduction of adipose tissue formation. The level of this reduction could be monitored, and if it becomes apparent after sufficient monitoring that the reduction is too low and modified as necessary by replacing the combined effects of the AED epimers, with βAED alone which would mediate a milder reduction in adipose tissue formation. If reduction is still too much, the effect could be corrected by administration of αAED after a sufficient period of time, which would up-regulate PPARγ and replenish the number of desired adipocytes. The particular permutation, dosage, and time period between doses used by the practitioner would enable achieving the desired effects. The converse administration is also possible.

When administered as described herein, these active agents can also regulate the levels of COX-2 and NF-κB, thereby regulating inflammation. As noted above, αAED increases the levels of COX-2, whereas βAED results in only a mild elevation of COX-2, and sequential administration of αAED and βAED reduces the levels of COX-2 and is anti-inflammatory. Furthermore, αAED increases the levels of the proinflammatory nuclear factor, NF-κB, whereas βAED reduces the levels of NF-κB. This divergence in mechanisms allows treatment to be tailored to the individual patient, by adjusting the amounts of each compound depending on the inflammatory disorder that the patient has.

According to the present invention, βAED or αAED (or analogs thereof) is administered in sufficient dosages to provide a blood concentration of about 5 to 10,000 nM of αAED or βAED. In general, an αAED dose of about 0.5 to about 300 mg/day for about 1 to about 4 days, usually about 5-250 mg/day will be suitable and efficacious for human therapeutic applications. αAED at a concentration of at least about 40 nM (e.g., about 40-200 nM), and usually about 50 nM is sufficient to regulate inflammation.

When sequential administration of compounds is used, the second compound is generally administered at least about 4-60 days after the last day on which the first compound was administered, e.g., about 7, 10, 14, 21, 28, 60, 90, 120, 150 or 180 days after administration of the last dose. In some embodiments, the first compound is administered once or twice within a period of about 2 to about 14 days, followed about 7 to about 21 days later by administration of the second compound daily for about 1 to about 10 days, using any of the dosages or dose ranges described herein. In other embodiments, the second compound is generally administered at least about 30 minutes, 1 hr, 2 hrs, 4 hrs, 6 hrs, 12 hrs, 24 hrs, 48 hrs, 72 hrs, 96 hrs, 120 hrs, 1 week, 2 weeks, 3 weeks, 1 month, 1.5 months, 2 months, 2.5 months, 3 months, 3.5 months, or 4 months after to the administration of the first compound.

The daily dosage of βAED is generally about 25-300 mg/day, usually about 40-150 mg/day (e.g., 30, 60, 80, 100, 120, or 150 mg/day), for human therapeutic applications. Doses of αAED or βAED are usually administered as a single dose, but may also be administered as two or more subdivided doses.

αAED and βAED (and their analogs) are administered by any conventional method, systemic or local. Suitable routes of administration include parenteral; topical; oral; rectal (e.g., suppositories, solutions for use as retention enemas and creams or jellies); buccal, sublingual (e.g., tablets or lozenges), intranasal or endotracheal (e.g., sprays, aerosols, or mists for inhalation or insufflation), mucosal, ocular, subcutaneous, and the like.

In some embodiments, αAED is administered locally. Alternatively, αAED is administered by applicator.

In a preferred embodiment, βAED is administered transepithelially, *e.g.*, in a manner that brings the compound into contact with an epithelial layer in the patient. Preferred transepithelial routes of administration include topical, ocular (instillation into the eye), intranasal, inhalation, insufflation, buccal, perioral, subcutaneous, rectal, sublingual, and any other mucosal route of administration.

The carrier system or vehicle used in a given instance will depend on the mode of administration. The active agents are lipophilic compounds. Solvents and carriers for lipophilic steroids known in the art are appropriate for use in the compositions containing βAED or αAED or analogues thereof. Examples of such carriers or vehicles are glycols such as polypropylene glycol, polyethylene glycol, ethanol, DMSO and cyclodextrins (especially the amorphous cyclodextrins). Cyclodextrins will pass through the buccal mucosa into the circulation easily. Other suitable vehicles that should be considered include fatty acid esters of polyoxyethylene sorbitol (Tweens) or sorbitan (Spans) for preparation of emulsions.

βAED or αAED may be delivered to or through the skin by any means, including subcutaneous or intradermal injection or topical application. One means of topical application is the use of skin patches impregnated with the active agent. This means of delivery is advantageous since it is non-invasive and easily administered by relatively unskilled care providers.

βAED and αAED can be formulated according to known methods to prepare pharmaceutically useful compositions such as by admixture with a pharmaceutically acceptable vehicle. Suitable vehicles and their formulation are described, for example, in Remington's Pharmaceutical Sciences (16th Ed., Osol, A. Ed., Mack Easton PA (1980)). To form a pharmaceutically acceptable composition suitable for effective administration, such compositions will contain an effective amount of βAED or αAED, either alone or with one or more additional active agents, and a suitable amount of carrier vehicle.

Additionally, and as will be appreciated by one of ordinary skill in the art, conventional additives and excipients will be useful in formulating pharmaceutical compositions comprising the active agents described herein. Such excipients include buffers, antioxidants, stabilizers, diluents, and the like. Those additives and excipients will be dictated by the mode of administration selected.

For transdermal administration, patches for administration of βAED or αAED may be formulated as adhesive patches containing the active agent. For example, the patch may be a discoid in which a pressure-sensitive silicone adhesive matrix containing the active agent may be covered with a non-permeable backing. The discoid may either contain the active agent in the adhesive or may be attached to a support made of material such as polyurethane foam or gauze that will hold the active agent. In all instances, the area to which the patch is applied should be cleaned carefully before application. Patches may be prepared containing permeation enhancers such as cyclodextrin, butylated hydroxyanisole, or butylated hydroxytoluene.

For application directly to the skin, βAED or αAED may, for example, be dissolved in carrier material containing DMSO and alcohol, then applied to a patch or directly to epidermal tissues. For rectal administration, βAED or αAED may be administered by suppository, enema, or by application of creams, etc. Compositions of the invention may be administered by any method that will result in contact of the active agent with tissue of ectodermal origin.

The βAED or αAED may be administered to the mucosa of oral, pharyngeal or nasal cavity by tablet or lozenge. When the active agent is administered to the mucosa of the oral cavity, it may be administered as a buccal tablet or spray or use in the oral-pharyngeal cavity and the nasal cavities.

When βAED or αAED or their analogues are administered orally, the active agents may be utilized more efficiently if the active agents are protected from destruction and absorption in the upper gastrointestinal tract. The active agents are most effective when the period of exposure to the mucosa of the intestinal tract is increased. Hence use of capsules containing the active agents in formulations that effect slow release in the intestine. Use of retention enemas for treatment is appropriate when the patient may have difficulty retaining compositions administered by mouth.

Compositions of the invention for use in installation into wounds, for injection into tissues or for application to operative areas may be prepared by solubilizing the active agents of the invention in agents such as lipid solvents (for example, hydroxypropylene glycol) used to solubilize steroidal materials. Because the active agents are effective upon encountering tissue of ectodermal origin, they may, for example, be administered directly into the operative area as a spray or installation during surgical treatment of prostate malignancies. Compositions such as liposomes, cyclodextrin inclusion complexes or impregnated polymeric materials (including naturally occurring polymers or synthetic polymers) are particularly useful for such application.

The following examples illustrate preferred embodiments of the invention; they should not be construed as limiting its scope in any way.

### Example 1

Dunning R3327, AT-1, rat prostate cancer, is a completely hormone-refractory tumor cell line. The AT-1 is a fast growing tumor, with a doubling time of 60 hours and displays a completely anaplastic growth pattern, but it is rarely metastatic (<5%). It lacks measurable quantities of receptor for androgens and estrogens. The clinical behavior of this tumor is not influenced by presence or absence of sex steroids (Isaacs et al, Prostate 9:261-281 (1986)).

After an initial passage in young male rats, the tumor was implanted dorsally into young intact (not castrated) male Copenhagen-Fischer rats (Copenhagen, Denmark) of approximately 250 grams. Tumor transplants were allowed to grow until palpable and visible and reached a mean size of 10mm in diameter prior to treatment. The vehicle for the drug was Polyethylene glycol 400 and ethanol 1:1.
The drug was injected adjacent to the tumor. Four experimental groups with 6 animals in each group were used:

Group 1: injected with a single dose of 80 mg each of Δ⁵-androstene-3β,17β-diol (Sigma Chemicals) .

Group 2 and 3: injected with a single dose of 10 mg of Δ⁵-androstene-3β,17α-diol, (Steraloid Inc.)

Group 4 served as untreated, tumor-bearing controls.

After 96 hours group 3, which had been treated with 10 mg of Δ⁵-androstene-3β,17α-diol, received a single injection of 80 mg of Δ⁵-androstene-3β,17β-diol in the same way as group 1.

In group 2 the experiment was terminated at 96 hours and for the remaining groups after 19 days due to excessive tumor growth in some of the rats. Animals were killed by carbondioxide and tumors were excised. Samples were taken in the periphery of each tumor, mounted on a marked cork and frozen in liquid nitrogen.

### Protein blotting:

Thawed tumors were homogenized in ice cold lysis-buffer (160mM NaCl, 10mM HEPES, 2mM CaCl 2.5% SDS, 0.5% Triton X-100, 100 µg/ml phenylmetylsulfonyl fluoride, 1 µg/ml leupeptin and 2 µg/ml aprotinin), placed on ice for 15 minutes and following clarification centrifuged for 10 minutes at 13,000 g. Protein content of homogenates were determined by Lowry assay Q.

Protein in lysates (90pg) were separated by electrophoresis using 8% SDS-PAGE, the isolated proteins were transferred onto a nitrocellulose membrane (Amersham) in a trans-blot electrophoretic transfer cell (Bio-Rad Laboratories). After trans-blotting and blocking with non-fat milk in TTBS (lx TTBS: 20mM Tris, 150mM NaCl, pH 7.5, 0.1% Tween-20) blots were probed with mouse monoclonal IgG1 PPARγ antibody (Santa Cruz Biotechnology), diluted 1:400 in TTBS containing 3%(w/v) non-fat dried milk. After washing in TTBS, the blots were reincubated with horseradish peroxidase conjugated secondary antibody (goat anti- mouse antibodies, Santa Cruz) diluted 1:4000 in TTBS containing 3% (w/v) non-fat, dried milk for one hour. Blots were developed using an enhanced chemiluminescence system (ECL, Amersham) and exposed to Hyperfilm ECL (Amersham). Optical density was measured densitometrically.

All experiments were repeated twice in separate assays.

Western blots using triplicate samples from each of the treatment groups were analyzed for Bropirimine (as a control), PPARγ, COX-2 and NF-κB levels. The level of PPARγ, COX-2 and NF-κB in untreated control samples was set at 100 R.A.U.

Treatment for 96 hours with αAED resulted in an elevation of PPARγ to a mean value of 190. In contrast, examination of tumor samples at 19 days after treatment with a single dose of βAED showed a reduced level of PPARγ to a mean value of 47. Tumor samples derived from animals exposed to the sequential treatment of αAED and βAED had a marked reduction of PPARγ to 20. Bropirimine treated tumor samples yielded PPARγ levels of 84.

Treatment for 96 hours with αAED resulted in an elevation of COX-2 to a mean value of 203. Examination of tumor samples at 19 days after treatment with a single dose of βAED also showed an elevated level of COX-2 to a mean value of 135. Tumor samples derived from animals exposed to the sequential treatment of αAED and βAED had a reduction of COX-2 to 80. Bropirimine treated tumor samples yielded COX-2 levels of 196.

Examination of tumor samples at 19 days after treatment with a single dose of βAED showed a reduced level of NF-κB to a mean value of 68 ± 13.6.

## Claims

1. Use of a compound of formula (I) or a compound of formula (II): 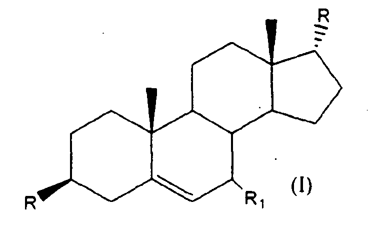 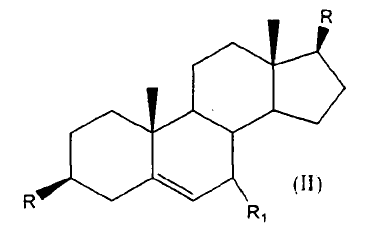 wherein each R₁ is H or R; and
wherein each R is independently selected from a hydroxyl, a protected hydroxyl, a C₁-C₃₀ ether and a C₁-C₃₀ ester;
for the manufacture of a medicament for regulating adipogenesis, angiogenesis, mesenteric fat hypertrophy, atherosclerosis or Alzheimer's disease.

2. Use according to claim 1, wherein each R is -OH.

3. Use according to claim 1 or claim 2, wherein each R₁ is -H.

4. Use according to any preceding claim, wherein the medicament is administered orally, topically, subcutaneously, parenterally, transdermally, mucosally, rectally, intranasally, via inhalation, via insufflation, via a patch, via application to the site of the tumor or tumor bed, via installation into a wound, by buccal, or by sublingual administration.

5. Use according to any preceding claim, wherein the medicament regulates nuclear transcription.

6. Use according to any preceding claim, wherein the medicament regulates the activity of PPAR-γ.

## Patentansprüche

1. Verwendung einer Verbindung gemäß Formel (I) oder einer Verbindung gemäß Formel (II) 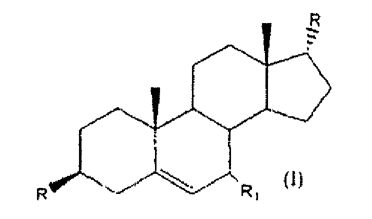 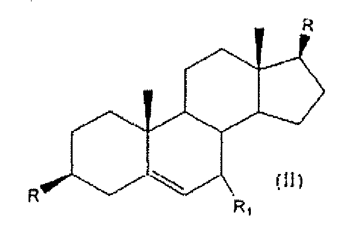 worin jeder Rest R₁ für H oder R steht; und
worin jeder Rest R unabhängig gewählt ist aus einem Hydroxyl-Rest, einem geschützten Hydroxyl-Rest, einem C₁- bis C₃₀-Ether-Rest und einem C₁- bis C₃₀-Ester-Rest;
für die Herstellung eines Medikaments zum Regulieren der Adipogenese, Angiogenese, mesenterischen Fett-Hypertrophie, Arteriosklerose oder Alzheimer-Krankheit.

2. Verwendung nach Anspruch 1, worin jeder Rest R für -OH steht.

3. Verwendung nach Anspruch 1 oder Anspruch 2, worin jeder Rest R₁ für -H steht.

4. Verwendung nach irgendeinem vorangehenden Anspruch, worin das Medikament verabreicht wird auf oralem, topischem, subkutanem, parenteralem, transdermalem, mukosalem, rektalem, intranasalem Weg, über Inhalation, über Insufflation, über ein Pflaster, über Aufbringung auf die Stelle des Tumors oder auf das Tumorbett, über Einbringung in eine Wunde, durch bukkale oder durch sublinguale Verabreichung.

5. Verwendung nach irgendeinem vorangehenden Anspruch, worin das Medikament die nukleare Transkription reguliert.

6. Verwendung nach irgendeinem vorangehenden Anspruch, worin das Medikament die Aktivität von PPAR-γ reguliert.

## Revendications

1. Utilisation d'un composé de formule (I) ou d'un composé de formule (II) : 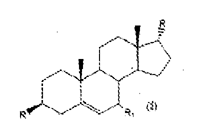 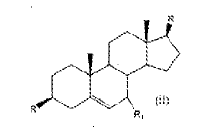 Où chaque R₁ est H ou R ; et
Où chaque R est indépendamment choisi parmi le groupe hydroxyle, un hydroxyle protégé, un éther C₁-C₃₀ et un ester C₁-C₃₀;
pour la fabrication d'un médicament en vue de réguler l'adipogénèse, l'angiogénèse, l'hypertrophie de graisse mésentérique,
l'arthérosclérose ou les maladies d'Alzheimer.

2. Utilisation selon la revendication 1, **caractérisée en ce que** chaque R est -OH.

3. Utilisation selon la revendication 1 ou la revendication 2, **caractérisée en ce que** chaque R₁ est - H.

4. Utilisation selon l'une des revendications précédentes, **caractérisée en ce que** le médicament est administré oralement, de manière topique, sous cutanée, parentérale, de manière transdermique, mucosale, rectale, intra-nasale, par inhalation, par insuflation, par « patch », par application sur la zone de tumeur ou le lit de la tumeur, par mise en place dans une blessure, par administration buccale ou sublinguale.

5. Utilisation selon l'une des revendications précédentes, **caractérisée en ce que** le médicament régule la transcription nucléaire.

6. Utilisation selon l'une des revendications précédentes, **caractérisée en ce que** le médicament régule l'activité de PPAR-_{γ}.
